# EUROPÄISCHE PATENTSCHRIFT

(11) **EP 3 675 917 B1**
(45) Veröffentlichungstag und Bekanntmachung des Hinweises auf die Patenterteilung: **31.03.2021**
(21) Anmeldenummer: 19805595.6
(22) Anmeldetag: 13.11.2019
(51) Int. Cl.: A61L 2/26, F16K 24/00, F16K 31/00

(54) **KONDENSATABLASSVENTIL**
CONDENSATE DRAIN VALVE
SOUPAPE D'ÉVACUATION DES CONDENSATS

(30) Priorität: 16.11.2018 DE 102018128871
(43) Veröffentlichungstag der Anmeldung: 08.07.2020
(73) Patentinhaber: Aesculap AG, 78532 Tuttlingen (DE)
(72) Erfinder: GRAY-DREIZLER, John, 78078 Niedereschach (DE); HENKE, Dr. Matthias, 78048 Vilingen-Schwenningen (DE); ELISCH, Andreas, 78655 Dunningen (DE); THOMAS, Stefan, 78532 Tuttlingen (DE)
(74) Vertreter: Winter, Brandl - Partnerschaft mbB
(86) Internationale Anmeldenummer: PCT/EP2019/081107
(87) Internationale Veröffentlichungsnummer: WO 2020/099455

(56) Entgegenhaltungen:
- EP-B1- 2 366 410
- DE-A1-102015 110 419
- DE-C1- 3 838 099
- DE-C2- 4 111 077
- DE-U1- 7 640 905
- DE-U1- 8 532 892

## Beschreibung

Die vorliegende Erfindung betrifft ein Kondensatablassventil für einen gasdicht verschließbaren Sterilcontainer.

### Hintergrund der Erfindung

Sterilcontainer der vorliegenden Gattung werden u.a. zur Sterilisation medizinischer Instrumente eingesetzt. Hierfür hat der Sterilcontainer eine Aufnahme- oder Containerwanne, in die zu sterilisierende Instrumente, das Sterilgut, eingelegt werden können, einen Containerdeckel zum fluiddichten Verschließen des Sterilcontainers sowie einen Verschluss/Verschlussmechanismus (Verriegelungsmechanismus), mittels welchem der Deckel mit der Containerwanne fest (fluiddicht) verriegelt werden kann.

Des Weiteren weist der Sterilcontainer üblicherweise einem Ventilmechanismus auf, der das Ansteigen eines Container-Innendrucks über einen den Sterilcontainer ggf. beschädigenden vorbestimmten/vorbestimmbaren Wert während eines Sterilisationsprozesses in einem Autoklav verhindert. Darüber hinaus ist es bekannt, dass der Ventilmechanismus derart ausgebildet/ ausgerüstet sein kann, dass durch den Ventilmechanismus hindurch Kondensat, welches sich während des Sterilisationsprozesses in dem Sterilcontainer ansammelt, ausgetragen werden kann.

### Stand der Technik

Aus dem Stand der Technik, beispielsweise gemäß einschlägiger Produkte der vorliegenden Anmelderin selbst, sind (medizinische) Sterilcontainer der vorstehend beschriebenen Gattung bekannt, die eine mittels eines Containerdeckels verschließbare Containerwanne sowie einen Verschluss-/Verriegelungsmechanismus haben, über welchen der Deckel mit der Wanne fluiddicht verriegelbar ist. Im Containerdeckel ist ferner eine Ventileinrichtung vorgesehen, die ein Einströmen von Umgebungsgas in den Containerinnenraum über eine (entkeimende) Filtereinrichtung und eine Ausströmung von Container-Innenraumgas in die Umgebung unter Umgehung der Filtereinrichtung erlaubt. Bei den bekannten Sterilcontainern ist vorgesehen, dass das Kondensat am Ende des Sterilisationsprozesses in eine definierte Trocknungsphase in die Gasform überführt und beispielsweise durch ein Filtermaterial des Sterilcontainers ausgebracht wird.

Weitere aus dem Stand der Technik bekannte Ventileinrichtungen sind beispielsweise in der DE 41 11 077 C2 und der EP 2 366 410 B1 beschrieben. Die Ventileinrichtungen weisen jeweils einen Aktuator/ Stellglied auf, mittels dessen bei Erreichen einer Zieltemperatur und/ oder eines Zieldrucks in einem den Aktuator/ Stellglied umgebenden Gasvolumen ein Ventilkörper verlagert und so ein Kondensatauslass freigegeben wird. Als besonders vorteilhaft haben sich hierbei Ventileinrichtungen erwiesen, die ein sogenanntes Absolutdruckventil aufweisen.

Als wichtig hat sich bei der Ausgestaltung von Ventileinrichtungen erwiesen, dass
- eine Beeinträchtigung eines Nutzraums des Sterilcontainer möglichst klein ist,
- eine Öffnung des Ventils ausschließlich während des Sterilisationsprozesses ermöglicht ist und
- Wartung, Montage und Funktionskontrolle der Ventileinrichtung möglichst einfach sind.

Darüber hinaus wäre es wünschenswert, dass
- die Ventileinrichtung von außen, ohne dass der Sterilcontainer geöffnet werden muss, überprüft werden kann,
- durch die Ventileinrichtung ausgebrachtes Kondensat nicht auf eventuell unter dem Sterilcontainer angeordnete weitere Sterilcontainer läuft und
- das Kondensat bereits während des Sterilisationsprozesses aus dem Sterilcontainer ausgetragen werden kann.

### Kurzbeschreibung der Erfindung

Angesichts vorstehender Beschreibung des Stands der Technik ist es die Aufgabe der vorliegenden Erfindung, eine funktionale Ventileinrichtung (Kondensatablassventil) für einen / eines Sterilcontainer(s) vorzugsweise der medizinischen Bauart bereitzustellen, mittels welchem die vorstehend genannten wünschenswerten Eigenschaften möglichst sämtlich erreichbar sind.

Diese Aufgabe wird durch eine Ventileinrichtung (Kondensatablassventil)für einen / eines vorzugsweise medizinischen Sterilcontainer(s) mit den Merkmalen des Patentanspruchs 1 bzw. durch einen (medizinischen) Sterilcontainer mit einer solchen Ventileinrichtung (Kondensatablassventil) gelöst. Vorteilhafte Ausgestaltungen/Weiterbildungen der Erfindung sind Gegenstand der Unteransprüche.

Die vorliegende Erfindung betrifft demzufolge ein Kondensatablassventil zur Anordnung (Montage) in einer Ventilaufnahme eines gasdicht verschließbaren Sterilcontainers, mit einem Ventilgehäuse, das zumindest einen Einlassabschnitt, der zu einem von dem Sterilcontainer umschlossenen Containergasvolumen hin eröffnet/ eröffenbar ist und zumindest einen Auslassabschnitt, der zu einem den Sterilcontainer zumindest abschnittsweise umgebenden Außengasvolumen hin eröffnet/ eröffenbar ist, aufweist, einem Bypass, durch den der Einlassabschnitt und der Auslassabschnitt mittels eines von dem Bypass abschnittsweise umschlossenen Bypassgasvolumens zumindest gas- und/ oder fluidleitend miteinander verbunden/ verbindbar sind, wobei in beanspruchter Weise das Bypassgasvolumen stets mit zumindest dem Containergasvolumen oder dem Außengasvolumen in Wirkverbindung steht, einem zwischen einer Sperrposition und einer Durchlassposition verlagerbaren Ventilkörper, durch den in seiner Sperrposition ein in der Ventilaufnahme angeordneter Kondensatauslass des Sterilcontainers verschließbar/ verschlossen ist und zumindest einem dem Ventilkörper zugeordneten und zumindest abschnittsweise von dem Bypassgasvolumen umgebenen Aktuator, durch den der Ventilkörper in Abhängigkeit von einer Temperatur und/ oder einem Druck des Bypassgasvolumens zwischen der Sperrposition und der Durchlassposition verlagert wird/ verlagerbar ist. Erfindungsgemäß weist das Ventilgehäuse mindestens einen (eine Art Scharnier bildenden) Haltesteg und mindestens ein von dem Haltesteg beabstandetes (Rastfeder-artiges) Verschlussmittel auf, wobei der Haltesteg zumindest abschnittsweise in einer Haltelasche/-öse der Ventilaufnahme anordenbar/ angeordnet ist und das Ventilgehäuse um eine parallel zu dem Haltesteg ausgerichtete (theoretische) Schwenkachse herum schwenkbar an der Ventilaufnahme festlegbar/ festgelegt ist, und das Ventilgehäuse um die Schwenkachse herum von einer Öffnungsposition in eine Einbauposition, in der das Verschlussmittel an einer Verschlussaufnahme des Sterilcontainers festgelegt/ festlegbar ist, verschwenkbar, ist der Ventilkörper bei in die Einbauposition überführtem Ventilgehäuse durch den an einer Aktuatoraufnahme des Ventilgehäuses festgelegten Aktuator in die Sperrposition überführt und mit einer Verschlusskraft beaufschlagt ist. Der Ventilkörper wird hierbei in bevorzugter Weise durch den Aktuator in die Öffnungsposition überführt, sobald das Bypassvolumen einen Öffnungsdruck und/ oder eine Öffnungstemperatur aufweist. Durch eine derartige Ausgestaltung des erfindungsgemäßen Kondensatablassventils wird erreicht, dass insbesondere während eines Sterilisationsprozesses ein Mediumsaustausch zwischen dem Containergasvolumen und dem Außengasvolumen ermöglicht ist und so Kondensat aus dem Sterilcontainer ausgetragen werden kann.

Demnach ist die Verlagerung des Ventilkörpers durch den Aktuator unabhängig von Druck- und/ oder Temperaturverhältnissen, die zwischen dem Containergasvolumen und dem Außengasvolumen ausgebildet sind, sondern lediglich von dem Druck und/ oder der Temperatur, dem/der der Aktuator durch das ihn zumindest abschnittsweise umgebende Bypassgasvolumen ausgesetzt ist. Sobald das Bypassgasvolumen eine Zieltemperatur und/ oder einen Zieldruck aufweist, bringt der Aktuator eine Stellkraft auf, verlagert den Ventilkörper von dem Kondensatauslass weg und öffnet so den Bypass. Eine bevorzugte Richtung des Mediumsaustauschs ist, entsprechend der grundlegenden Gesetzmäßigkeiten der Strömungsmechanik, abhängig von den in den Volumina herrschenden Bedingungen. Bei einem Überdruck im Containergasvolumen findet der Mediumsaustausch von dem Containergasvolumen in Richtung des Außengasvolumens statt. Bei eine einem Unterdruck im Containergasvolumen findet der Mediumsaustausch von dem Außengasvolumen in Richtung des Containergasvolumens statt. Falls das Kondensatablassventil bei Gleichdruck zwischen den Gasvolumina geöffnet ist, unterliegt der Mediumsaustausch den Gesetzmäßigkeiten der Diffusion.

Dadurch, dass das Ventilgehäuse dem Erfindungsgedanken folgend von der Einbauposition (Montage-Position) in die Öffnungsposition (Demontage-Position) verschwenkbar ist, kann das erfindungsgemäße Kondensatablassventil auf einfache Weise gereinigt, kontrolliert und/ oder instandgesetzt werden.

Um eine Dichtwirkung zwischen dem Ventilkörper und dem Kondensatauslass zu erhöhen, ist es bevorzugt vorgesehen, dass der Ventilkörper Dichtelemente, insbesondere O-Ringe, Flachdichtungen oder Ähnliches, aufweisen kann. Darüber hinaus ist es auch möglich und vorgesehen, dass der Kondensatauslass einen Ventilsitz aufweisen kann, der so an den Ventilkörper angepasst ist, dass zwischen dem Ventilkörper und dem Ventilsitz bei in die Sperrposition überführtem Ventilkörper eine durchgehende Dichtfläche ausgebildet ist, in der der Ventilkörper und der Ventilsitz dichtend in Anlage gebracht sind. Zur Verbesserung der Dichtwirkung zwischen Ventilkörper und Ventilsitz kann der Ventilsitz Dichtelemente aufweisen.

Um eine besonders einfache Montage des Aktuators zu ermöglichen, ist es vorgesehen, dass der Aktuator mittels einer Schnappverbindung an dem Ventilgehäuse festgelegt werden kann. Um ein ungewolltes Umströmen des Aktuators zumindest zu minimieren und bevorzugt zu verhindern, ist es darüber hinaus vorteilhafterweise vorgesehen, dass der Aktuator zusätzlich mittels eines adhäsiven Festlegeverfahrens, insbesondere unter Verwendung eines dichtenden Klebstoffs, an der Aktuatoraufnahme festgelegt ist.

Es ist demgegenüber jedoch auch möglich, dass der Aktuator lösbar an der Aktuatoraufnahme festgelegt sein kann. Geeignete lösbare Verbindungen sind beispielsweise Schraubverbindungen, Steckverbindungen, Rastverbindungen, Verbindungen nach Art eines Bajonettverschlusses oder Ähnliche.. Bei einem derart ausgestalteten Kondensatablassventil ist insbesondere vorteilhaft, dass einzelne Teile des Kondensatablassventils, insbesondere der Ventilkörper und der Aktuator, ausgetauscht und/ oder erneuert werden können.

Um eine Abdichtung zwischen dem Aktuator und der Aktuatoraufnahme zu verbessern, ist es auch möglich, dass zwischen dem Aktuator und der Aktuatoraufnahme ein Dichtelement, beispielsweise ein O-Ring, eine Dichtlippe oder Ähnliches, angeordnet und/ oder ausgebildet sein kann

Bei einer vorteilhaften Umsetzung des Erfindungsgedankens ist vorgehen, dass der Aktuator einen Faltenbalg aufweist/ als ein Faltenbalg ausgebildet ist, von dem ein Aktuatorgasvolumen umschlossen und hermetisch gegenüber dem Bypassgasvolumen abgedichtet ist, sodass der Faltenbalg in Abhängigkeit von einer zwischen dem Bypassgasvolumen und dem Aktuatorgasvolumen ausgebildeten Druckdifferenz axial gespreizt oder gestaucht wird. Faltenbalg im Sinne des Erfindungsgedankens meint ein im Wesentlichen zylindrisches Konstruktionselement, dass bevorzugt um eine Längsachse herum rotationssymmetrisch aufgebaut ist, wobei auf einer Mantelfläche des Faltenbalgs zumindest eine einen ersten Bereich der Mantelfläche von einem zweiten Bereich der Mantelfläche abgrenzende Falte angeordnet ist, durch die ein zwischen den Mantelflächen ausgebildeter Winkel veränderbar ist, bzw. die Mantelflächen um die Falte herum gegeneinander verkippbar sind, sodass ein Abstand zwischen einer ersten Deckfläche des Faltenbalgs und einer zweiten Deckfläche des Faltenbalgs veränderbar ist, der Faltenbalg also gestreckt und/ oder gestaucht werden kann. Bevorzugt weist der Faltenbalg eine Vielzahl von Falten auf.

Da der erfindungsgemäß vorgesehene Faltenbalg des Aktuators ein vorzugsweise hermetisch abgeschlossenes Aktuatorgasvolumen umschließt, ist vorgesehen, dass zum Strecken und/ oder Stauchen des Faltenbalgs ein Druck des den Aktuator zumindest abschnittsweise umgebende Bypassgasvolumens derart verändert werden muss, dass das Aktuatorgasvolumen in Relation zu dem Bypassgasvolumen zumindest einen Unterdruck aufweist. Darüber hinaus ist es vorgesehen, dass das Aktuatorgasvolumen, solange es in Relation zu dem Bypassgasvolumen einen Überdruck aufweist, mittels des Faltenbalgs zumindest anteilig zu der durch den Aktuator auf den Ventilkörper ausgeübten Verschlusskraft beiträgt.

Bei einer vorteilhaften Ausgestaltung des Kondensatablassventils ist vorgesehen, dass der Aktuator ein Federelement/ einen Federabschnitt aufweist, durch das/ den die Spreizung und/ oder Stauchung des Faltenbalgs unterstützt ist. Durch eine derartige Ausgestaltung des Aktuators wird es ermöglicht, dass mechanische Eigenschaften des Aktuators angepasst werden können. Durch das Federelement/ den Federabschnitt kann beispielsweise die Verschlusskraft unabhängig von weiteren Parametern, insbesondere Druck und/ oder Temperatur des Bypassgasvolumens eingestellt werden. Es ist auch möglich und vorgesehen, dass das Federelement/ der Federabschnitt eine veränderliche Federkonstante aufweisen kann, sodass eine auf den Aktuator/ den Faltenbalg wirkende Federvorspannung verändert werden kann. Es ist dabei vorgesehen, dass die Federvorspannung so eingestellt sein kann, dass sowohl das Stauchen, als auch das Strecken des Faltenbalgs unterstützt sein können.

Es ist vorgehsehen, dass das Federelement beispielsweise innerhalb des Faltenbalgs angeordnet sein kann und insbesondere auf die Deckflächen des Faltenbalgs wirkt. Bei einer besonders vorteilhaften Ausführungsform ist es vorgesehen, dass der Federabschnitt durch die Mantelflächen und/ Falten des Faltenbalgs gebildet ist, beispielsweise indem lokal eine Materialstärke und damit eine Steifigkeit erhöht ist.

Eine vorteilhafte Ausgestaltung der Erfindung sieht vor, dass der Aktuator ein mechanisch auf den Aktuator wirkendes Bimetallelement aufweist, durch das insbesondere die Verschlusskraft in Abhängigkeit von einer Temperatur des Bypassgasvolumens veränderbar ist. Durch eine derartige Ausgestaltung des Aktuators wird erreicht, dass die Verschlusskraft nicht lediglich von dem in dem Bypassgasvolumen herrschenden Druck abhängig ist. Hierdurch ist es ermöglicht, dass die vor, während und nach dem Sterilisationsprozess durch den Aktuator auf den Ventilkörper ausgeübte Verschlusskraft auch in Abhängigkeit zu einer Temperatur, insbesondere der in dem Bypassgasvolumen herrschenden Temperatur, gesetzt sein kann. Dies ist insbesondere vorteilhaft, wenn in und um den Sterilisationscontainer ausgebildete Druckverhältnisse als alleiniger Parameter zur Regelung eines Dichtverhaltens des Kondensatablassventils während des Sterilisationsprozesses nur unzureichend geeignet sind.

Um insbesondere einen unkomplizierten Ein- und Ausbau des Kondensatablassventils zu unterstützen, ist es erfindungsgemäß vorgesehen, dass das Verschlussmittel zumindest einen federbedrückten Rasthaken aufweist, durch den bei in die Einbauposition überführtem Ventilgehäuse ein als Rastnase ausgebildeter Abschnitt der Verschlussaufnahme zumindest abschnittsweise hintergriffen ist. Bevorzugt weist der Rasthaken eine rampenartig ausgebildete Führungsfläche auf, die beim Überführen des Ventilgehäuses von der Öffnungsposition in die Einbauposition an der Rastnase entlanggleitet, wobei der Rasthaken entgegen eines auf ihn wirkenden Federdrucks verlagert wird bis die Einbauposition erreicht ist und der Rasthaken durch den Federdruck derart verschoben wird, dass die Rastnase durch den Rasthaken hintergriffen und das Ventilgehäuse mittels des Rasthakens und der Rastnase zumindest im Bereich von Rasthaken und Rastnase an der Ventilaufnahme festgelegt ist. Darüber hinaus ist eine Vielzahl weiterer rastender Verbindungen bekannt, die allesamt geeignet sind als Kombination aus Verschlussmittel und Verschlussaufnahme des erfindungsgemäßen Kondensatablassventils verwendet zu werden.

Um eine einfache Bedien- und Lösbarkeit der aus Rasthaken und Rastnase bildbaren/ gebildeten Verbindung zu erreichen, ist es bevorzugt vorgesehen, dass der Rasthaken mit einem Bedienelement in Wirkverbindung gebracht ist, durch das der Rasthaken von der Rastnase weg verlagerbar ist, sodass mittels des Bedienelements eine zwischen dem Rasthaken und der Rastnase ausgebildete form- und/ oder kraftschlüssige Verbindung lösbar ist. Besonders bevorzugt ist das Bedienelement als ein Schiebeschalter ausgebildet, der mit dem Rasthaken direkt oder über eine Mechanik in Wirkverbindung gebracht ist. Es ist auch möglich, dass das Bedienelement als ein Druckschalter ausgebildet sein kann, der mit einer Umlenkmechanik in Wirkverbindung gebracht ist, wobei durch die Umlenkmechanik eine auf den Schalter ausgeübte Betätigungskraft derart umgelenkt wird, dass die eingangs beschriebene Verlagerung des Rasthakens ermöglicht ist.

Weiter alternativ kann es vorgesehen sein, dass das Verschlussmittel ein erstes Festlegeelement und die Verschlussaufnahme ein zweites Festlegelement aufweist und die Festlegeelemente derart aneinander angepasst sind, dass bei in die Einbauposition überführtem Ventilgehäuse mittels der Festlegeelemente eine Schraubverbindung herstellbar ist. Bevorzugt weist das derartig ausgestaltete Verschlussmittel eine Schraubenausnehmung zum Durchführen eines Schafts einer Schraube und die Verschlussaufnahme eine an die Schraube angepasste Gewindeausnehmung mit einem Innengewinde auf, wobei die Schraubenausnehmung und die Gewindeausnehmung bei in die Einbauposition überführtem Ventilgehäuse in Deckung gebracht sind und die Schraube in das Innengewinde der Gewindeausnehmung eingeschraubt werden kann, sodass das Ventilgehäuse durch einen in einem Randbereich der Schraubenausnehmung mit dem Verschlussmittel in Anlage gebrachten Schraubenkopf der Schraube an der Ventilaufnahme festgelegt/ festlegbar ist. Darüber hinaus ist es auch möglich, dass die Schraube verliersicher an dem Verschlussmittel angeordnet sein kann. Der Schraubenkopf kann eine Vielzahl unterschiedlicher Schraubenkopfantriebe aufweisen, beispielsweise Innensechskant, Innensechsrund, Außensechskant, Kreuzschlitz, Schlitz oder Ähnliche.

Eine vorteilhafte Umsetzung des Erfindungsgedankens sieht vor, dass das Ventilgehäuse ein sich von dem Einlassabschnitt bis zu einem Containerboden des Sterilcontainers erstreckendes Ansaugrohr aufweist, durch das bei in die Durchlassposition überführtem Ventilkörper und einem Überdruck des Containergasvolumens in Relation zu dem Außengasvolumen durch das Ansaugrohr Kondensat durch Einlassabschnitt, Bypass, Auslassabschnitt und Kondensatauslass aus dem Sterilcontainer herausbeförderbar ist. Durch eine derartige Ausgestaltung des Kondensatventils wird erreicht, dass das Kondensat durch das Ansaugrohr sowohl hydropneumatisch, als auch unter Ausnutzung des Ejektoreffekts gefördert werden kann. Bei der hydropneumatischen Förderung ist vorgesehen, dass eine im Bereich des Containerbodens angeordnete Ansaugöffnung vollständig in das Kondensat eingetaucht ist und in dem Containergasvolumen ein auf das Kondensat wirkender Überdruck herrscht. Die Förderung unter Ausnutzung des Ejektoreffekts beruht darauf, dass aus dem Containergasvolumen ausströmendes Gas, insbesondere Dampf, im Bereich der Ansaugöffnung angesammeltes Kondensat "mitreißt" und so aus dem Sterilcontainer fördert, sodass auch kleiner Mengen Kondensat gefördert werden können, die bei rein hydraulischer Förderung nicht aus dem Sterilcontainer ausgebracht werden könnten.

Eine vorteilhafte Ausgestaltung der Erfindung sieht vor, dass das Kondensatablassventil zumindest abschnittsweise auf einer auf dem Containerboden aufstehenden Containerwandung angeordnet ist. Durch eine derartige Anordnung des Kondensatablassventil ist eine Einschränkung eines in dem Sterilcontainer für ein Sterilgut zur Verfügung stehenden Nutzraums minimiert. Bei einer besonders vorteilhaften Ausführungsform der Erfindung ist vorgesehen, dass das Kondensatablassventil zumindest teilweise in die Containerwandung eingelassen ist, sodass das Kondensatablassventil so wenig als möglich von der Containerwandung hervorspringt und so vor Beschädigung durch das Sterilgut, insbesondere während eines Transports, geschützt ist.

Bei einer vorteilhaften Ausgestaltung des erfindungsgemäßen Kondensatablassventils ist vorgesehen, dass der Ventilkörper einen bei in die Einbauposition überführtem Ventilgehäuse und in die Sperrposition überführtem Ventilkörper durch den Kondensataustritt hindurch sichtbaren/ fühlbaren/ wahrnehmbaren Kontrollstift aufweist, sodass ein bestimmungsgemäßer Einbau und/ oder eine bestimmungsgemäße Funktion des Kondensatablassventils werkzeugfrei ohne einen Zugang von dem Containergasvolumen her überprüfbar ist. Durch eine derartige Ausgestaltung des Kondensatablassventils ist es ermöglicht, dass ein Nutzer vor Beginn des Sterilisationsprozesses überprüfen kann, ob das Kondensatablassventil bestimmungsgemäß verbaut ist. Darüber hinaus ist nach dem Sterilisationsprozess überprüfbar, ob das Kondensatablassventil ordnungsgemäß verschlossen ist, sodass bestimmt werden kann, ob eine Sterilität des Sterilguts gegeben ist.

Die Erfindung betrifft auch einen Sterilcontainer mit einem Kondensatablassventil der eingangs beschriebenen Art.

### Kurzbeschreibung der Figuren

Die Erfindung wird nachstehend anhand bevorzugter Ausführungsbeispiele unter Bezugnahme auf die begleitenden Figuren näher erläutert.
Fig. 1 zeigt einen Querschnitt eines in einen Sterilcontainer eingesetzten Kondensatablassventils,
Fig. 2 zeigt ein Kondensatablassventil wie es in eine Containerwandung eines Sterilcontainers eingesetzt ist,
Fig. 3 zeigt wie ein Kondensatablassventil von der Einbauposition in die Öffnungsposition überführbar ist,
Fig. 4 und 5 zeigen unterschiedliche Ansichten eines ausgebautes Kondensatablassventils,
Fig. 6 zeigt ein Kondensatablassventil mit entnommenem Aktuator und
Fig. 7 zeigt eine Detailansicht eines Kondensatauslasses eines Sterilcontainers mit eingebauten Kondensatablassventil.

### Figurenbeschreibung

In den Figuren ist eine bevorzugte Ausführungsform eines erfindungsgemäßen Kondensatablassventils 1 zur Verwendung mit einem Sterilcontainer 2 gezeigt, wobei der dargestellte Sterilcontainer 2 zwecks einer Verbesserung der Übersichtlichkeit ohne einen Containerdeckel und Sterilgut dargestellt ist. Das Kondensatablassventil 1 ist in einer Ventilaufnahme 4 des Sterilcontainers 2 angeordnet und an dieser festgelegt. Das Kondensatablassventil 1 weist ein Ventilgehäuse 6 auf, das einen Haltesteg 8 und ein von dem Haltesteg 8 beabstandetes Verschlussmittel 10 aufweist. Der Haltesteg 8 ist abschnittsweise in einer Haltelasche 12 der Ventilaufnahme 4 angeordnet. Das Ventilgehäuse 6 ist um eine parallel zu dem Haltesteg 8 (theoretische) ausgerichtete Schwenkachse 14 herum schwenkbar an der Ventilaufnahme 4 festgelegt. Das Ventilgehäuse 6 ist um die Schwenkachse 14 herum von einer Öffnungsposition in eine Einbauposition verschwenkbar. Das Ventilgehäuse 6 des dargestellten Kondensatablassventils 1 ist in die Einbauposition verschwenkt, in der das Verschlussmittel 10 an einer Verschlussaufnahme 16 des Sterilcontainers 2 festgelegt ist.

Das Ventilgehäuse 6 weist einen Einlassabschnitt 18 und einen Auslassabschnitt 20 auf. Der Einlassabschnitt 18 des Ventilgehäuses 6 ist zu einem Containergasvolumen 22 eröffnet, wobei der Auslassabschnitt 20 mittels eines Kondensatauslasses 24 des Sterilcontainers 2 zu einem den Sterilcontainer 2 umgebenden Außengasvolumen 26 hin eröffnet ist. Darüber hinaus weist das Ventilgehäuse 6 einen sich durch das Ventilgehäuse 6 erstreckenden Bypass 28 auf. Durch den Bypass 28 ist ein Bypassgasvolumen 30 umschlossen. Mittels des Bypassgasvolumens 30 sind der Einlassabschnitt 18 und der Auslassabschnitt 20 gas- und fluidleitend miteinander verbunden/ verbindbar. Das Bypassgasvolumen 30 steht bei der dargestellten Ausführungsform des Kondensatablassventils 1 stets mit dem Containergasvolumen 22 in Wirkverbindung.

In dem Bypass 28 ist ein zwischen einer Sperrposition und einer Durchlassposition verlagerbaren Ventilkörper 32 angeordnet. Dem Ventilkörper 32 ist ein Aktuator 34, der an einer Aktuatoraufnahme 36 des Ventilgehäuses 6 festgelegt ist, zugeordnet. Dadurch, dass das Ventilgehäuse 6 in die Einbauposition überführt ist, ist der Ventilkörper 32 durch den Aktuator 34 mit einer Verschlusskraft 38 beaufschlagt und in die Sperrposition überführt und verschließt den Kondensatauslass 24.

Der Aktuator 34 ist als ein Faltenbalg ausgebildet und abschnittsweise von dem Bypassgasvolumen 30 umgeben. Da das Bypassgasvolumen 30 in Wirkverbindung mit dem Containergasvolumen 22 steht, sind die in den Volumina 22, 33 ausgebildeten Drücke zumindest direkt proportional voneinander abhängig. Während eines Sterilisationsprozesses steigt die Temperatur des Containergasvolumens 22 und somit, in Folge des hermetischen Abschlusses des Sterilcontainers 2, auch sein Druck. Aufgrund der direkten Wirkverbindung zwischen dem Containergasvolumen 22 und dem den Aktuator 34 umgebenen Bypassgasvolumen 22, steigt auch der Druck in dem Bypassgasvolumen 22. Durch den Aktuator 34 ist ein Aktuatorgasvolumen 40 umschlossen und hermetisch gegenüber dem Bypassgasvolumen 30 abgedichtet. Sobald der Druck des Bypassgasvolumens 30 einen Öffnungsdruck übersteigt wird der als Faltenbalg ausgebildete Aktuator 34 gestaucht und auf den Ventilkörper 32 wird eine der ursprünglich auf den Ventilkörper 32 wirkenden Verschlusskraft 38 entgegen gerichtete Stellkraft 42 ausgeübt, durch die der Ventilkörper 32 von der Sperrposition in die Durchlassposition überführt wird. Es ist vorgesehen, dass der Öffnungsdruck beispielsweise dadurch eingestellt werden kann, dass ein in dem hermetisch abgeschlossenen Aktuatorgasvolumen 40 ausgebildeter Druck verringert oder erhöht wird. Je höher der Druck im Aktuatorgasvolumen 40 ist, umso höher muss der Druck im Bypassgasvolumen 30 sein, damit der Faltenbalg gestaucht werden kann.

Um eine möglichst kompakte Bauform zu realisieren, ist die dargestellte Ausführungsform des Kondensatablassventil 1 auf einer auf einem Containerboden 44 des Sterilcontainers 2 aufstehenden Containerwandung (Seitenwand) 46 angeordnet.

Das Ventilgehäuse 6 des dargestellten Kondensatablassventils 1 weist ein sich von dem Einlassabschnitt 18 bis zu dem Containerboden 44 des Sterilcontainers 2 erstreckendes Ansaugrohr (Schnabel) 48 auf. Durch das Ansaugrohr 48 ist bei in die Durchlassposition überführtem Ventilkörper und einem Überdruck des Containergasvolumens 22 in Relation zu dem Außengasvolumen 26 insbesondere Kondensat aus dem Sterilcontainer 2 austragbar, das sich am Containerboden 44 angesammelt hat.

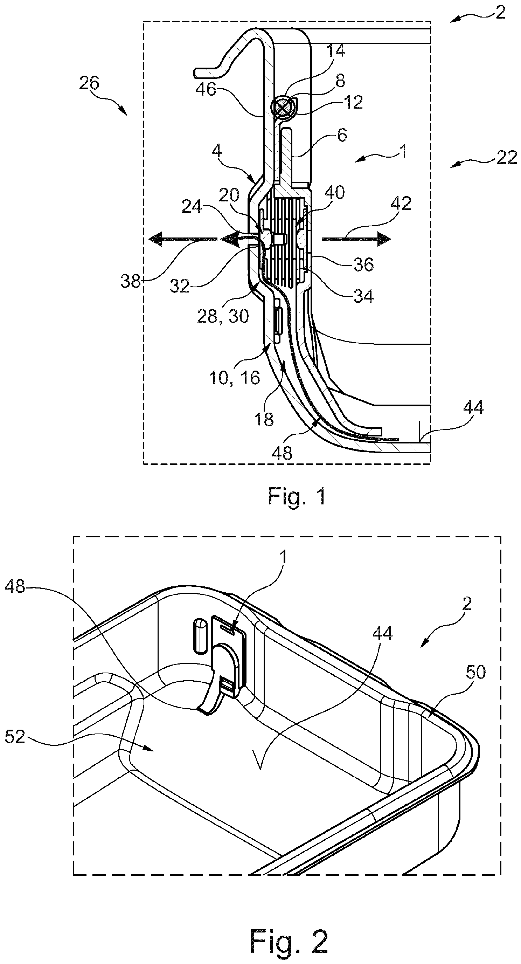

In Figur 2 ist das in einer Containerwanne 50 des Sterilcontainers 2 angeordnete Kondensatablassventil 1 in der Einbaulage gezeigt. Die Containerwanne 50 weist im Bereich des Containerbodens 44 eine Kondensatsammelrinne 52 auf, die sich bis zu dem Ansaugrohr 48 des Kondensatablassventils erstreckt und durch die ein Sammeln des Kondensats im Bereich des Ansaugrohrs 48 begünstigt ist.

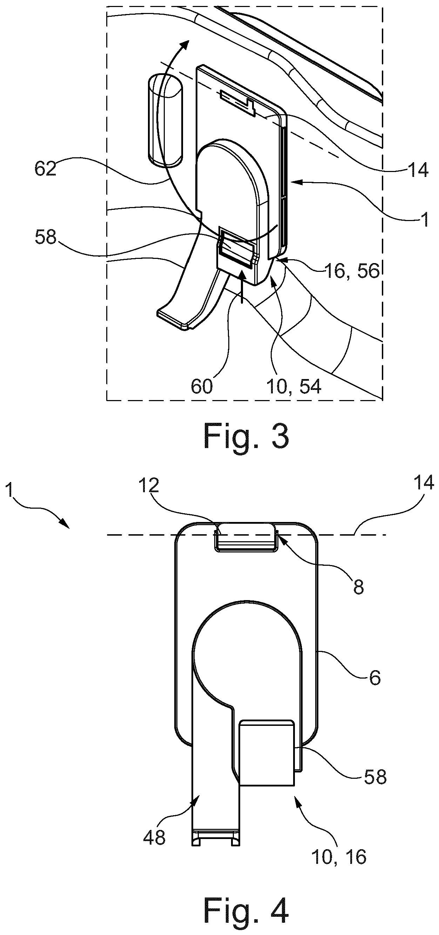

In Figur 3 ist eine vergrößerte Ansicht des bereits in Figur 2 gezeigten Kondensatablassventils 1 gezeigt. Die Figur zeigt, wie das in der Einbauposition angeordnete Kondensatablassventil 1 in die Öffnungsposition überführt werden kann. Das Verschlussmittel 10 weist einen federbedrückten Rasthaken 54 auf, durch den ein als Rastnase 56 ausgebildeter Abschnitt der Verschlussaufnahme 16 abschnittsweise hintergriffen ist. Der Rasthaken 54 ist mit einem als Schiebeschalter ausgebildeten Bedienelement 58 in Wirkverbindung gebracht. Sobald auf das Bedienelement 58 eine Betätigungskraft 60 ausgeübt wird, wird der Rasthaken 54 von der Rastnase 56 weg verlagert und die Verbindung zwischen Rasthaken 54 und Rastnase 56 gelöst. Das Ventilgehäuse 6 ist dann um die Schwenkachse 14 herum, entlang eines Bogens 62 von der Einbauposition in die Öffnungsposition verlagerbar.

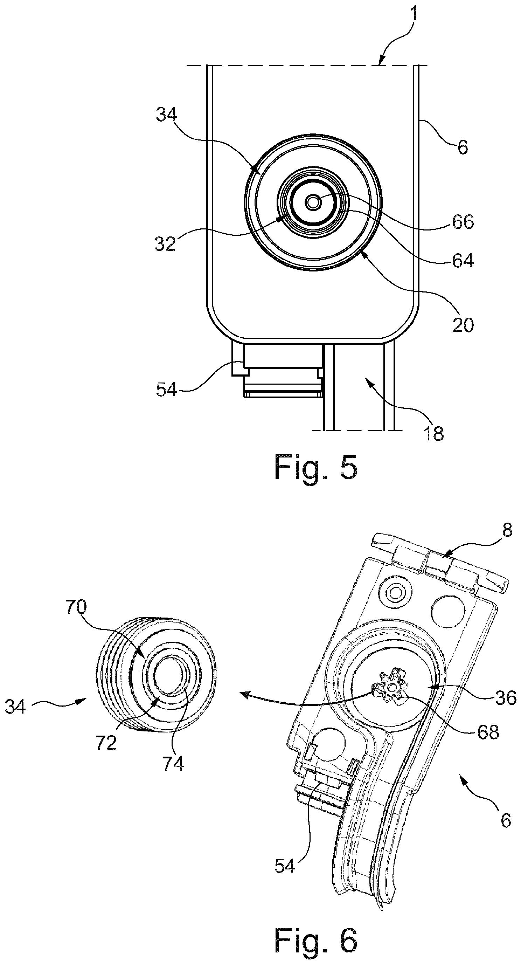

In den Figuren 4 und 5 ist das Kondensatablassventil 1 in einander entgegengerichteten Draufsichten gezeigt. In Figur 5 ist das Ventilgehäuse 6 mit dem Ventilkörper 32 und einem an dem Ventilkörper festgelegten Dichtelement 64 gezeigt, welches bei in die Sperrposition überführtem Ventilkörper 32 mit einem Umlaufend um den nicht dargestellten Kondensatauslass angeordneten Dichtbereich in Anlage gebracht ist. In der Mitte des Ventilkörpers 32 ist ein Kontrollstift 66 angeordnet, der bei in die Sperrposition überführtem Ventilkörper 32 durch den Kondensatauslass herausragt. In der Figur sind ebenfalls der Einlassabschnitt 18, der Auslassabschnitt 20, der Aktuator 34 und der Rasthaken 54 gezeigt.

Bei dem in Figur 6 dargestellten und aus dem Sterilcontainer 2 ausgebauten Kondensatablassventil 1, ist der Aktuator 34 von dem Ventilgehäuse 6 getrennt. Das Ventilgehäuse 6 weist im Bereich der Aktuatoraufnahme 36 drei in einer Umfangsrichtung äquidistant zueinander angeordnete Schnapphaken 68 auf. Der Aktuator 34 weist in einem Festlegebereich 70 einen umlaufenden Vorsprung 72 auf, der bei bestimmungsgemäß in die Aktuatoraufnahme 36 eingesetztem Aktuator 34 durch die Schnapphaken 68 hintergriffen ist. Zum Festlegen des Aktuators 34 an dem Ventilgehäuse 6 wird der Aktuator 34 mit dem Festlegebereich 70 voran derart in die Aktuatoraufnahme 36 eingesetzt, dass die Schnapphaken 68 an dem Vorsprung 72 vorbeigeführt und zumindest abschnittsweise mit einer dem Außengasvolumen 26 zugewandten Anlagefläche 74 in Anlage gebracht sind. Wenn der Aktuator 34 bestimmungsgemäß in die Aktuatoraufnahme 36 eingesetzt ist, dann ist zwischen dem Aktuator 34 und dem Ventilgehäuse 6 mittels der Schnapphaken 68, dem Vorsprung 72 und der Anlagefläche 74 in dem Festlegebereich 70 eine Schnappverbindung ausgebildet. In der Figur sind zur Verdeutlichung nochmals der Rasthaken 54 und der Haltesteg 8 gezeigt.

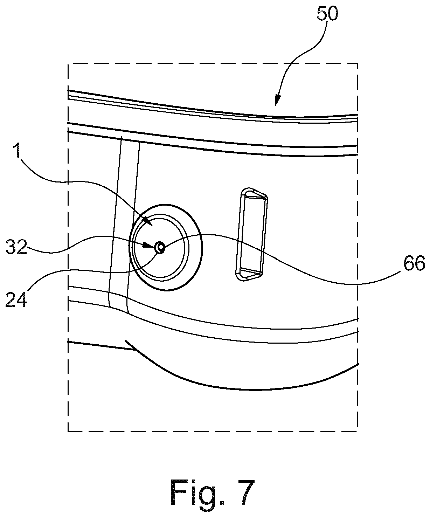

In Figur 7 ist eine von außen auf die Containerwanne 50 mit einem in die Ventilaufnahme 4 eingesetzten Kondensatablassventil 1 gezeigt. Der Kontrollstift 66 ragt durch den Kondensatauslass 24 hindurch, sodass erkennbar ist, dass das Kondensatablassventil 1 bestimmungsgemäß eingebaut und der Ventilkörper 32 in die Sperrposition überführt ist.

### Bezugszeichenliste

- 1.: Kondensatablassventil
- 2.: Sterilcontainer
- 4.: Ventilaufnahme
- 6.: Ventilgehäuse
- 8.: Haltesteg
- 10.: Verschlussmittel
- 12.: Haltelasche
- 14.: Schwenkachse
- 16.: Verschlussaufnahme
- 18.: Einlassabschnitt
- 20.: Auslassabschnitt
- 22.: Containergasvolumen
- 24.: Kondensatauslass
- 26.: Außengasvolumen
- 28.: Bypass
- 30.: Bypassgasvolumen
- 32.: Ventilkörper
- 34.: Aktuator
- 36.: Aktuatoraufnahme
- 38.: Verschlusskraft
- 40.: Aktuatorgasvolumen
- 42.: Stell kraft
- 44.: Containerboden
- 46.: Containerwandung
- 48.: Ansaugrohr
- 50.: Containerwanne
- 52.: Kondensatsammelrinne
- 54.: Rasthaken
- 56.: Rastnase
- 58.: Bedienelement
- 60.: Betätigungskraft
- 62.: Bogen
- 64.: Dichtelement
- 66.: Kontrollstift
- 68.: Schnapphaken
- 70.: Festlegebereich
- 72.: Vorsprung
- 74.: Anlagefläche

## Patentansprüche

1. Kondensatablassventil (1) zur Anordnung in einer Ventilaufnahme (4) eines gasdicht verschließbaren Sterilcontainers (2), mit
- einem Ventilgehäuse (6), das zumindest einen Einlassabschnitt (18), der zu einem von dem Sterilcontainer (2) umschlossenen Containergasvolumen (22) hin eröffnet/ eröffenbar ist und zumindest einen Auslassabschnitt (20), der zu einem den Sterilcontainer (2) zumindest abschnittsweise umgebenden Außengasvolumen (26) hin eröffnet/ eröffenbar ist, aufweist,
- einem Bypass (28), durch den der Einlassabschnitt (18) und der Auslassabschnitt (20) mittels eines von dem Bypass (28) abschnittsweise umschlossenen Bypassgasvolumens (30) zumindest gas- und/ oder fluidleitend miteinander verbunden/ verbindbar sind und das Bypassgasvolumen (30) stets mit zumindest dem Containergasvolumen (22) oder dem Außengasvolumen (26) in Wirkverbindung steht,
- einem zwischen einer Sperrposition und einer Durchlassposition verlagerbaren Ventilkörper (32), durch den in seiner Sperrposition ein in der Ventilaufnahme (4) angeordneter Kondensatauslass (24) des Sterilcontainers (2) verschließbar/ verschlossen ist und
- zumindest einem dem Ventilkörper (32) zugeordneten und zumindest abschnittsweise von dem Bypassgasvolumen (30) umgebenen Aktuator (34), durch den der Ventilkörper (32) in Abhängigkeit von einer Temperatur und/ oder einem Druck des Bypassgasvolumens (30) zwischen der Sperrposition und der Durchlassposition verlagert wird/ verlagerbar ist,
**dadurch gekennzeichnet, dass**
- das Ventilgehäuse (6) mindestens einen Haltesteg (8) und mindestens ein von dem Haltesteg (8) beabstandetes Verschlussmittel (10) aufweist, wobei der Haltesteg (8) zumindest abschnittsweise in einer Haltelasche (12) der Ventilaufnahme (4) anordenbar/ angeordnet ist und das Ventilgehäuse (6) um eine parallel zu dem Haltesteg (8) ausgerichtete Schwenkachse (14) herum schwenkbar an der Ventilaufnahme (4) festlegbar/ festgelegt ist,
- das Ventilgehäuse (6) um die Schwenkachse (14) herum von einer Öffnungsposition in eine Einbauposition verschwenkbar ist, in der das Verschlussmittel (10) an einer Verschlussaufnahme (16) des Sterilcontainers (2) festgelegt/ festlegbar ist,
- der Ventilkörper (32) bei in die Einbauposition überführtem Ventilgehäuse (6) durch den an einer Aktuatoraufnahme (36) des Ventilgehäuses (6) festgelegten Aktuator (34) in die Sperrposition überführt und mit einer Verschlusskraft (38) beaufschlagt ist und
- der Ventilkörper (32) durch den Aktuator (34) in die Öffnungsposition überführt wird, sobald das Bypassgasvolumen (30) einen Öffnungsdruck und/ oder eine Öffnungstemperatur aufweist, sodass ein Mediumsaustausch zwischen dem Containergasvolumen (22) und dem Außengasvolumen (26) ermöglicht ist.

2. Kondensatablassventil (1) nach Anspruch 1, **dadurch gekennzeichnet, dass** der Aktuator (34) einen Faltenbalg aufweist/ als ein Faltenbalg ausgebildet ist, von dem ein Aktuatorgasvolumen (40) umschlossen und hermetisch gegenüber dem Bypassgasvolumen (30) abgedichtet ist, sodass der Faltenbalg in Abhängigkeit von einer zwischen dem Bypassgasvolumen (30) und dem Aktuatorgasvolumen (40) ausgebildeten Druckdifferenz axial gespreizt oder gestaucht wird.

3. Kondensatablassventil (1) nach Anspruch 2, **dadurch gekennzeichnet, dass** der Aktuator (34) ein Federelement/ einen Federabschnitt aufweist, durch das/ den die Spreizung und/ oder Stauchung des Faltenbalgs unterstützt ist.

4. Kondensatablassventil (1) nach einem der Ansprüche 1 bis 3, **dadurch gekennzeichnet, dass** der Aktuator (34) ein mechanisch auf den Aktuator (34) wirkendes Bimetallelement aufweist, durch das insbesondere die Verschlusskraft (38) in Abhängigkeit von einer Temperatur des Bypassgasvolumens (30) veränderbar ist.

5. Kondensatablassventil (1) nach einem der Ansprüche 1 bis 4, **dadurch gekennzeichnet, dass** das Verschlussmittel (10) zumindest einen federbedrückten Rasthaken (54) aufweist, durch den bei in die Einbauposition überführtem Ventilgehäuse (6) ein als Rastnase (56) ausgebildeter Abschnitt der Verschlussaufnahme (16) zumindest abschnittsweise hintergriffen ist.

6. Kondensatablassventil (1) nach Anspruch 5, **dadurch gekennzeichnet, dass** der Rasthaken (54) mit einem Bedienelement (58) in Wirkverbindung gebracht ist, durch das der Rasthaken (54) von der Rastnase (56) weg verlagerbar ist, sodass mittels des Bedienelements (58) eine zwischen dem Rasthaken (54) und der Rastnase (56) ausgebildete form- und/ oder kraftschlüssige Verbindung lösbar ist.

7. Kondensatablassventil (1) nach einem der Ansprüche 1 bis 6, **dadurch gekennzeichnet, dass** das Verschlussmittel (10) ein erstes Festlegeelement und die Verschlussaufnahme (16) ein zweites Festlegelement aufweist und die Festlegeelemente derart aneinander angepasst sind, dass bei in die Einbauposition überführtem Ventilgehäuse (6) mittels der Festlegeelemente eine Schraubverbindung herstellbar ist.

8. Kondensatablassventil (1) nach einem der Ansprüche 1 bis 7, **dadurch gekennzeichnet, dass** das Ventilgehäuse (6) ein sich von dem Einlassabschnitt (18) bis zu einem Containerboden (44) des Sterilcontainers (2) erstreckendes Ansaugrohr (48) aufweist, durch das bei in die Durchlassposition überführtem Ventilkörper (32) und einem Überdruck des Containergasvolumens (22) in Relation zu dem Außengasvolumen (26) durch das Ansaugrohr (48) Kondensat durch Einlassabschnitt (18), Bypass (28), Auslassabschnitt (20) und Kondensatauslass (24) aus dem Sterilcontainer (2) herausbeförderbar ist.

9. Kondensatablassventil (1) nach einem der Ansprüche 1 bis 8, **dadurch gekennzeichnet, dass** das Kondensatablassventil (1) zumindest abschnittsweise auf einer auf dem Containerboden (44) aufstehenden Containerwandung angeordnet ist.

10. Kondensatablassventil (1) nach einem der Ansprüche 1 bis 9, **dadurch gekennzeichnet, dass** der Ventilkörper (32) einen bei in die Einbauposition überführtem Ventilgehäuse (6) und in die Sperrposition überführtem Ventilkörper (32) durch den Kondensatauslass (24) hindurch sichtbaren/ fühlbaren/ wahrnehmbaren Kontrollstift (66) aufweist, sodass ein bestimmungsgemäßer Einbau und/ oder eine bestimmungsgemäße Funktion des Kondensatablassventils (1) werkzeugfrei ohne einen Zugang von dem Containergasvolumen (22) her überprüfbar ist.

11. Sterilcontainer (2) mit einem Kondensatablassventil (1) nach einem der Ansprüche 1 bis 10.

## Claims

1. Condensate drain valve (1) for arrangement in a valve mounting (4) of a sterile container (2) that can be closed in a gastight manner, comprising
- a valve housing (6), which has at least one inlet portion (18), which is opened/openable towards a container gas volume (22) enclosed by the sterile container (2), and at least one outlet portion (20), which is opened/openable towards an external gas volume (26) that at least partially surrounds the sterile container (2),
- a bypass (28), by means of which the inlet portion (18) and the outlet portion (20) are connected/connectable to one another in at least gas and/or fluid conducting fashion by means of a bypass gas volume (30) that is partially enclosed by the bypass (28), and the bypass gas volume (30) is always in operative connection with at least the container gas volume (22) or the external gas volume (26),
- a valve body (32) which can be displaced between a blocking position and a passage position and by means of which, in its blocking position, a condensate outlet (24) of the sterile container (2) arranged in the valve mounting (4) is closeable/closed and
- at least one actuator (34) associated with the valve body (32) and at least partially surrounded by the bypass gas volume (30), by means of which the valve body (32) is displaced/displaceable between the blocking position and the passage position depending on a temperature and/or a pressure of the bypass gas volume (30),
**characterised in that**
- the valve housing (6) has at least one retaining bar (8) and at least one closure means (10) spaced apart from the retaining bar (8), wherein the retaining bar (8) can be arranged/is arranged at least partially in a retaining lug (12) of the valve mounting (4) and the valve housing (6) can be fixed/is fixed to the valve mounting (4) so as to be pivotable about a pivot axis (14) aligned parallel to the retaining bar (8),
- the valve housing (6) can be pivoted about the pivot axis (14) from an open position to an installation position in which the closure means (10) is fixed/can be fixed to a closure mounting (16) of the sterile container (2),
- with the valve housing (6) transferred to the installation position, the valve body (32) is transferred to the blocking position by the actuator (34) fixed to an actuator mounting (36) of the valve housing (6) and is subjected to a closing force (38), and
- the valve body (32) is transferred to the open position by the actuator (34) as soon as the bypass gas volume (30) has an opening pressure and/or an opening temperature, so that an exchange of medium between the container gas volume (22) and the outer gas volume (26) is made possible.

2. Condensate drain valve (1) according to claim 1, **characterised in that** the actuator (34) comprises/is configured as a bellows by which an actuator gas volume (40) is enclosed and hermetically sealed with respect to the bypass gas volume (30), so that the bellows is axially expanded or compressed in dependence on a pressure difference formed between the bypass gas volume (30) and the actuator gas volume (40).

3. Condensate drain valve (1) according to claim 2, **characterised in that** the actuator (34) has a spring element/spring portion by which the expansion and/or compression of the bellows is supported.

4. Condensate drain valve (1) according to any one of claims 1 to 3, **characterised in that** the actuator (34) has a bimetallic element which acts mechanically on the actuator (34) and by means of which in particular the closing force (38) can be varied in dependence on a temperature of the bypass gas volume (30).

5. Condensate drain valve (1) according to any one of claims 1 to 4, **characterised in that** the closure means (10) has at least one spring-loaded latching hook (54), by means of which, with the valve housing (6) transferred to the installation position, a portion of the closure mounting (16) configured as a latching nose (56) is at least partially engaged.

6. Condensate drain valve (1) according to claim 5, **characterised in that** the latching hook (54) is brought into operative connection with an operating element (58) by means of which the latching hook (54) can be displaced away from the latching nose (56), so that a form-fitting and/or force-fitting connection formed between the latching hook (54) and the latching nose (56) can be released by means of the operating element (58).

7. Condensate drain valve (1) according to any one of claims 1 to 6, **characterised in that** the closure means (10) has a first fixing element and the closure mounting (16) has a second fixing element and the fixing elements are adapted to one another in such a way that, with the valve housing (6) transferred to the installation position, a screw connection can be produced by means of the fixing elements.

8. Condensate drain valve (1) according to any one of claims 1 to 7, **characterised in that** the valve housing (6) has a suction pipe (48) extending from the inlet portion (18) to a container base (44) of the sterile container (2), by means of which, with the valve body (32) transferred to the passage position and an excess pressure of the container gas volume (22) in relation to the external gas volume (26), through the suction pipe (48), condensate can be conveyed through the inlet portion (18), bypass (28), outlet portion (20) and condensate outlet (24), out of the sterile container (2).

9. Condensate drain valve (1) according to any one of claims 1 to 8, **characterised in that** the condensate drain valve (1) is arranged at least partially on a container wall upstanding from the container floor (44).

10. Condensate drain valve (1) according to any one of claims 1 to 9, **characterised in that** the valve body (32) has, with the valve housing (6) transferred to the installation position and the valve body (32) transferred to the blocking position, a control pin (66) that can be seen/felt/perceived through the condensate outlet (24), so that proper installation and/or proper function of the condensate drain valve (1) can be checked without tools and without access from the container gas volume (22).

11. Sterile container (2) comprising a condensate drain valve (1) according to any one of claims 1 to 10.

## Revendications

1. Soupape d'évacuation de condensat (1) destinée à être montée dans un logement de réception de soupape (4) d'un récipient stérile pouvant être fermé de manière étanche aux gaz (2), comprenant
- un boîtier de soupape (6), qui présente au moins une partie d'entrée (18), qui s'ouvre/peut s'ouvrir en direction d'un volume de gaz de récipient (22) entouré par le récipient stérile (2) et au moins une partie de sortie (20), qui s'ouvre/peut s'ouvrir en direction d'un volume de gaz extérieur entourant au moins par endroits un récipient stérile (2),
- un bypass (28), par lequel la partie d'entrée (18) et la partie de sortie (20) sont reliés/peuvent être reliées entre elles au moins de manière à conduire le gaz/le liquide au moyen d'un volume de gaz de bypass (30) entouré par endroits par le bypass (28) et le volume de gaz de bypass (30) reste constamment en liaison active avec au moins le volume de gaz de récipient (22) ou le volume de gaz de sortie (26),
- un corps de soupape (32) pouvant être déplacé entre une position verrouillée et une position de passage, par lequel une ouverture de sortie de condensat (24) disposée dans un logement de réception de soupape (4) du récipient stérile (2) peut être fermée/est fermée dans sa position verrouillée et
- au moins un actionneur (34) associé au corps de soupape (32) et au moins entouré par endroits par le volume de gaz de bypass (30) par lequel le corps de soupape (32) est déplacé/peut être déplacé en fonction d'une température et/ou d'une pression du volume de gaz de bypass (30) entre la position verrouillée et la position de passage,
**caractérisée en ce que**
- le boîtier de soupape (6) présente au moins une nervure de retenue (8) et au moins un moyen de fermeture (10) espacé d'une nervure de retenue (8), la nervure de retenue (8) pouvant être disposée/étant disposée au moins par endroits dans un axe de support (12) du logement de réception de soupape (4) et le boîtier de soupape (6) pouvant être fixé/étant fixé autour d'un axe de pivotement (14) orienté parallèlement à la nervure de retenue (8) de manière à pouvoir pivoter au niveau du logement de réception de soupape (4),
- le boîtier de soupape (6) peut être pivoté autour de l'axe de pivotement (14) d'une position d'ouverture à une position de montage, dans lequel le moyen de fermeture (10) est fixé/peut être fixé au logement de fermeture (16) du récipient stérile (2),
- le corps de soupape (32) pour le boîtier de soupape (6) transféré dans la position de montage par lequel l'actionneur fixé à un logement d'actionneur (36) du boîtier de soupape (6) est transféré dans la position verrouillée et est sollicité avec une force de fermeture (38) et
- le corps de soupape (32) est transféré par l'actionneur (34) dans la position d'ouverture, dès que le volume de gaz de bypass (30) présente une pression d'ouverture et/ou une température d'ouverture, de sorte qu'un changement de milieu entre le volume de gaz de récipient (22) et le volume de gaz extérieur (26) est possible.

2. Soupape d'évacuation de condensat (1) selon la revendication 1, **caractérisée en ce que** l'actionneur (34) présente un soufflet/lorsqu'un soufflet est formé, entouré d'un volume de gaz d'actionneur (40) et est fermé hermétiquement par rapport au volume de gaz de bypass (30), de sorte que le soufflet est écarté ou écrasé axialement d'une différence de pression formée entre le volume de gaz de bypass (30) et le volume de gaz de l'actionneur (40).

3. Soupape d'évacuation de condensat (1) selon la revendication 2, **caractérisée en ce que** l'actionneur (34) présente un élément élastique/une partie de ressort, par lequel/laquelle l'écartement et/ou l'écrasement du soufflet est soutenu.

4. Soupape d'évacuation de condensat (1) selon l'une quelconque des revendications 1 à 3, **caractérisée en ce que** l'actionneur (34) présente un élément bimétallique agissant de manière mécanique sur l'actionneur (34), par lequel en particulier la force de fermeture (38) peut être modifiée en fonction d'une température du volume de gaz de bypass (30).

5. Soupape d'évacuation de condensat (1) selon l'une quelconque des revendications 1 à 4, **caractérisée en ce que** le moyen de fermeture (10) présente au moins un crochet d'encliquetage (54) comprimé par le ressort, par lequel pour le boîtier de soupape (6) transféré dans la position de montage, une partie formée en tant qu'ergot d'arrêt (56) du logement de fermeture (16) est au moins mis en prise par endroits.

6. Soupape d'évacuation de condensat (1) selon la revendication 5, **caractérisée en ce que** le crochet d'encliquetage (54) est amené en liaison active avec l'élément de commande (58), par lequel le crochet d'encliquetage (54) est écarté de l'ergot d'arrêt (56), de sorte qu'au moyen de l'élément de commande (58) une liaison de forme et/ou de force formée entre le crochet d'encliquetage (54) et l'ergot d'arrêt (56) peut être détachée.

7. Soupape d'évacuation de condensat (1) selon l'une quelconque des revendications 1 à 6, **caractérisée en ce que** le moyen de fermeture (10) présente un premier élément de fixation et le logement de fermeture (16) un second élément de fixation et les éléments de fixation sont ainsi adaptés les uns aux autres, **en ce qu'**un vissage peut être réalisé pour le boîtier de soupape (6) transféré dans la position de montage au moyen des éléments de fixation.

8. Soupape d'évacuation de condensat (1) selon l'une quelconque des revendications 1 à 7, **caractérisée en ce que** le boîtier de soupape (6) présente un tube d'aspiration (48) s'étendant de la partie d'entrée (18) jusqu'à un fond de récipient (44) du récipient stérile (2), par lequel pour le corps de soupape (32) transféré dans la position de passage et une suppression du volume de gaz de récipient (22) par rapport au volume de gaz de sortie (26) par le tube d'aspiration (48) le condensat peut être évacué par la partie d'entrée (18), le bypass (28), la partie de sortie (20) et l'orifice de sortie du condensat (24) du récipient stérile (2).

9. Soupape d'évacuation de condensat (1) selon l'une quelconque des revendications 1 à 8, **caractérisée en ce que** la soupape d'évacuation de condensat (1) est disposée au moins par endroits sur une paroi de récipient montée sur le fond de récipient (44).

10. Soupape d'évacuation de condensat (1) selon l'une quelconque des revendications 1 à 9, **caractérisée en ce que** le corps de soupape (32) présente pour un boîtier de soupape(6) transféré dans une position de montage et un corps de soupape (32) transféré dans la position verrouillée par la sortie du condensat (24) une goupille de contrôle (66) visible/tactile/perceptible, de sorte qu'une installation dans les conditions envisagées et/ou une fonction dans les conditions envisagées de la soupape d'évacuation de condensat (1) peut être vérifiée sans outil sans devoir accéder au volume de gaz de récipient (22).

11. Récipient stérile (2) comprenant une soupape d'évacuation de condensat (1) selon l'une quelconque des revendications 1 à 10.
